# FASCICULE DE BREVET EUROPEEN

(11) **EP 1 082 125 B1**
(45) Date de publication et mention de la délivrance du brevet: **24.04.2002**
(21) Numéro de dépôt: 99922248.2
(22) Date de dépôt: 02.06.1999
(51) Int. Cl.: A61K 35/78

(54) **UTILISATION PHARMACEUTIQUE OU COSMETIQUE D'UN EXTRAIT DE RUSCUS ACULEATUS POUR LE TRAITEMENT DES DESORDRES CAPILLAIRES**
PHARMAZEUTISCHE ODER KOSMETISCHE VERWENDUNG VON EINEM EXTRAKT VON RUSCUS ACULEATUS ZUR BEHANDLUNG VON HAARSTÖRUNGEN
PHARMACEUTICAL OR COSMETICAL USE OF AN EXTRACT OF RUSCUS ACULEATUS FOR THE TREATMENT OF HAIR DISORDERS

(30) Priorité: 04.06.1998 FR 9807022
(43) Date de publication de la demande: 14.03.2001
(73) Titulaire: Pierre Fabre Dermo-Cosmetique, 92100 Boulogne-Billancourt (FR)
(72) Inventeur: CHARVERON, Marie, F-31000 Toulouse (FR); FABRE, Bernard, F-31450 Belberaud (FR); JEANJEAN, Michel, F-31320 Castanet-Tolosan (FR); NAVARRO, Roger, F-09100 Pamiers (FR)
(74) Mandataire: Ahner, Francis
(86) Numéro de dépôt international: FR9901285
(87) Numéro de publication internationale: WO9962529

(56) Documents cités:
- FR-A- 2 104 911
- FR-A- 2 258 175
- GB-A- 1 380 253
- PATENT ABSTRACTS OF JAPAN vol. 096, no. 009, 30 septembre 1996 (1996-09-30) & JP 08 133950 A (SHISEIDO CO LTD), 28 mai 1996 (1996-05-28)

## Description

L'invention a pour objet l'utilisation d'un extrait de Ruscus aculeatus pour la fabrication d'une composition pharmaceutique ou cosmétique, destinée au traitement des désordres capillaires.

En ce qui concerne les désordres capillaires, ceux-ci peuvent être d'origine occasionnelle ou installée. Par désordres capillaires, on entend toute modification touchant à la structure ou au fonctionnement du follicule pileux. Plus particulièrement, par désordres capillaires, on entend toute perturbation biologique entraînant la perte occasionnelle ou prolongée des cheveux.

Depuis de nombreuses années, on s'est attaché à lutter contre les désordres touchant la croissance ou le maintien du cheveu et des structures dont il dépend.

Ces désordres capillaires peuvent avoir différentes causes d'origines internes ou externes.

Par origines internes, on entend toutes perturbations touchant les processus biologiques impliqués dans la croissance et le maintien du cheveu. On citera par exemple le métabolisme hormonal, le dérèglement sébacé, l'amplification enzymatique des 5-α-réductases, l'inflammation, les modifications structurales du collagène, l'intervention des facteurs de croissance, des facteurs nerveux ou des facteurs vasculaires et la composante génétique.

Par origines externes, on entend, l'incidence de la flore locale du cuir chevelu, l'action d'agents chimiques (colorations, permanentes, chimiothérapie), la pollution ou l'hygiène de vie.

Tous ces désordres, d'origines internes ou externes, ciblant le cuir chevelu, ont pour conséquence directe la perturbation et la modification du cycle pilaire conduisant à différentes formes d'alopécies.

Le terme d'alopécie résume l'ensemble des pathologies atteignant le follicule pileux, son environnement, ainsi que le cycle de croissance de la tige pilaire et ayant pour conséquence la perte occasionnelle et réversible ou la chute définitive de tout ou partie des cheveux.

On citera les 2 types d'alopécies les plus répandus, l'*Alopécie Aérata* et l'*Alopécie Androgénogénétique* ou *Androgénique.*

Depuis longtemps, les scientifiques recherchent à travers différentes voies biologiques, les solutions visant à stimuler la croissance du cheveu ou à prolonger son attachement au cuir chevelu. cette préoccupation est d'autant plus marquée dans le domaine de l'alopécie androgénique et plus particulièrement fréquente chez les sujets masculins, chez lesquels on constate une perturbation du cycle de croissance pilaire, entraînant la chute du cheveu.

Le follicule pileux constitue l'unité biologique fondamentale, directement impliquée dans le développement et le maintien du système pilaire. Il rassemble différentes entités structurales telles que la papille dermique, la glande sébacée, les gaines épithéliales, l'innervation ou la vascularisation impliquées séparément ou conjointement dans le cycle de développement des cheveux. Le lien existant entre la papille dermique et le bulbe reste déterminant dans la croissance du cheveu (cf. Oliver RF. Histological studies of whisker regeneration in the hooded rat. J Embryol Exp Morphol 1966; 311. 560-2 et Jahoda CAB. Home KA. Oliver RF. Induction of hair growth by implantation of cultured dermal papilla cells. Nature 1966 ; 311. 560-2.

Le cycle de croissance pilaire est divisé en trois phases principales :
- une PHASE ANAGENE active ou phase de croissance pendant laquelle le follicule s'enfonce dans les tissus dermiques. Les cellules du bulbe entament une mitose rapide suivie d'une différenciation. La phase anagène est reconnue aujourd'hui comme l'étape primordiale de la croissance du cheveu, son accélération expliquant le raccourcissement de la vie du cheveu et sa rapidité de chute.
- une PHASE CATAGENE au cours de laquelle les divisions cellulaires régressent puis s'arrêtent. A ce stade, le follicule pileux amorce une remontée extra-dermique et une rétraction, c'est la phase la plus courte estimée à environ 3 semaines.
- une PHASE DE REPOS OU PHASE TELOGENE pendant laquelle le cheveu est progressivement délogé par un nouveau cheveu anagène issu du même follicule. Le cheveu natif va provoquer la chute du cheveu mature.

Il existe une perte de cheveux normale et physiologique de l'ordre de 60 à 100 par jour pour une chevelure saine. Au-delà, la chute est dite pathologique qu'elle soit occasionnelle ou installée.

A ce jour, on a proposé différents produits pour lutter contre les deux types d'alopécies. La plupart associent plusieurs principes actifs susceptibles d'apporter une action bénéfique sur les paramètres biologiques mis en cause dans la chute des cheveux.

Parmi les principes actifs les plus couramment rencontrés, nous pouvons citer à titre d'exemples :
- des vitamines telles que les vitamines A, E, B5, B6, C, H, PP.
- des oligo-éléments tels que le zinc, le cuivre, le magnésium, le silicium,
- des dérivés protéiques tels que les peptides, les acides aminés soufrés (type méthionine, cystine, cestéïne ou dérivés),
- des huiles essentielles ou des extraits d'origine végétale dont la liste n'est pas limitative,
- des agents antifongiques tels que la piroctonolamine, les dérivés undécyléniques, la cyclopiroxolamine,
- des molécules de synthèse chimique réputées pour leur action spécifique au niveau des récepteurs androgéniques ou sur la synthèse ou l'expression des 5-α-réductases.

Parmi les dernières découvertes effectuées ces dernières années, le MINOXIDIL® (ou 2,4 diamino-6-pipéridinoperimidine 3-oxide) fait aujourd'hui référence dans le traitement de l'alopécie androgénique. En dépit des nombreuses théories évoquées sur son mécanisme d'action, ce dernier n'est pas clairement élucidé. De plus, son efficacité reste limitée, même s'il est constaté une stabilisation de la chute dans de nombreux cas cliniques en raison de la reprise du processus alopéciant dès l'arrêt du traitement. Son usage journalier contraignant est vraisemblablement à l'origine d'effets secondaires indésirables relevés chez des patients l'utilisant à long terme tels que réactions cutanées localisées ou effets systémiques.

Dernièrement, on a proposé dans les demandes de brevet WO 9501197 et WO 9201437 une nouvelle molécule inspirée de la précédente la 2,4 diamino-pyrimidine 3-oxide ou AMINEXIL®.

L'Aminexil agit contre la fibrose périfolliculaire en inhibant l'expression de la lysylhydroxylase, elle-même responsable de la transformation accélérée de l'hydroxylysine conduisant à la formation précoce de collagène mature. Cette surproduction de collagène et simultanément sa réticulation entraînent une rigidification de la gaine conjonctive et une fibrose périfolliculaire s'opposant aux processus de formation des néo-cheveux.

Tous ces différents traitements proposés restent insuffisants parce que ne s'attaquant bien souvent qu'à l'une des causes directement impliquées dans le phénomène de l'alopécie.

L'objet de la présente invention est de maintenir ou stimuler la croissance des cheveux exposés à une chute anormale comme c'est le cas dans les alopécies aérata ou androgéniques. Plus précisément la présente invention concerne l'action d'un extrait *Ruscus aculeatus* sur l'expression du VEGF ou facteur de croissance vasculaire endothélial ou « vascular endothelial growth factor ».

La papille dermique du follicule pileux, structure contrôlant la croissance pilaire, se caractérise par la présence d'un réseau vasculaire (cf. Richard A. Ellis, Guiseppe Moretti. Vascular patterns associated with catagen hair follicules in the human scalp. Annals New York Academy of Sciences, 1959-83, 448-457) très développé en phase anagène. Il a été démontré que les cellules de cette papille expriment durant ce stade et de façon intense, un facteur de croissance angiogénique le VEGF (S.Lachgar - M.Charveron in « Hair for the nex millenium »- EdS. D,JJ Van Neste - VA. Randall - H. Baden - H. Ogawa et R. Oliver - Elsevier - Amsterdam -1996 - p. 407-412).

Si la croissance du poil dépend de plusieurs facteurs, les facteurs susceptibles d'induire une angiogénèse sont des candidats de choix dans l'induction et le maintien de la vascularisation au niveau de la papille dermique. Cette découverte est d'autant plus importante qu'il y a disparition du réseau capillaire durant le passage du stade anagène au stade catagène. Ainsi, le développement pilaire semble être lié au maintien des capillaires sanguins puisque la chute du poil est associée à une disparition du réseau capillaire de la papille durant la phase télogène.

Il a également été démontré que les cellules de la papille dermique des follicules pileux présentent des récepteurs au facteur VEGF et le produisent in vitro (S. Lachgar - M. Charveron - J. Invest. Dermatol. 1996, 107-p. 17-23).

La présente invention concerne l'utilisation d'une composition pharmaceutique ou cosmétique capillaire, caractérisée en ce qu'elle comprend dans un véhicule approprié, une dose efficace d'un extrait de *Ruscus aculeatus* stimulant la production du facteur de croissance vasculaire endothélial (VEGF).

La production du facteur de croissance vasculaire endothélial (VEGF) peut être mesurée au moyen du test décrit ci-après et l'augmentation de la production peut être évaluée en comparant la composition selon l'invention à une composition témoin ne comprenant pas d'extrait végétal.

L'extrait de *Ruscus aculeatus* est évalué pour son activité stimulante dans le temps sur la cinétique de production de VEGF par les cellules en culture de la papille dermique folliculaire du cuir chevelu humain.

En effet, les cellules de la papille dermique du follicule pileux présentent des récepteurs à ce facteur et le produisent *in vitro*. La mise en évidence de l'activité de l'extrait végétal est effectuée selon la technique décrite ci-après à partir d'une solution d'extrait végétal à 8,9 % de matière active.

La suspension de cellules de la papille dermique (2.5 x 10⁵ cellules/ 2 ml) dans le milieu DMEM contenant du sérum de veau foetal (SVF) est distribuée dans les plaques 6 puits (2.5 x 10⁵ cellules/puits) et incubée pendant 18 heures à 37°C dans une atmosphère à 5 % CO₂. Les cellules de la papille folliculaire sont alors rincées avec un tampon phosphate PBS pour éliminer les cellules non-adhérentes puis exposées aux produits à tester inclus dans du milieu DMEM.

Les milieux de culture de chacun des puits sont récupérés à t + 12 h, t + 24h, t + 48h et t + 72h, centrifugés à 3000 tr/mn et conservés à -80°C.

Après avoir récupéré les surnageants, les cellules sont traitées durant la même cinétique avec du tampon d'extraction (1ml/puits) composé de Tris-HCI 10 mM, du MgCl₂ 2mM, du NaCI 150 mM, du Triton X100 à 1 % et du PMSF 2 mM (phénylméthylsulfonylfluoride).

Les lysats cellulaires sont ensuite récupérés dans des tubes, soniqués pendant 4 x 15 secondes puis centrifugés à 3000 t/min pendant 15 min à +4°C, cette étape étant nécessaire à l'élimination des débris cellulaires.

Les surnageants sont ensuite conservés à -80°C avant leur utilisation ultérieure pour le dosage du VEGF.

La production du VEGF dans les extraits cellulaires et dans les surnageants est mesurée par Kit Elisa (R&D Biosystems).

Les résultats sont exprimés en picogrammes de VEGF par ml de milieu et en pourcentages d'activité.

La présente invention concerne l'utilisation d'une composition cosmétique pour le traitement des cheveux caractérisée en ce que ledit extrait de *Ruscus aculeatus*, induit une augmentation de la production de VEGF par rapport au témoin d'au moins environ 10 %.

L'extrait de *Ruscus aculeatus* est notamment caractérisé en ce qu'il comprend une teneur efficace en hétérosides stéroliques totaux (HST).

Les HST représentent une famille assez étendue de substances. Le composé majoritaire est le desgluconéoruscoside accompagné de la néoruscine, de la dégluconéoruscine.

En général, les teneurs en HST se situent entre 8 et 40 %.

Le *Ruscus aculeatus* appelé communément Petit Houx est un sous-arbrisseau appartenant à la famille des liliacées. Son rhizome constitue la drogue végétale à partir de laquelle est préparé l'extrait selon la présente invention et selon les procédés d'extraction décrits ci-après.

Les rhizomes de Ruscus sont préalablement séchés puis broyés afin de permettre l'extraction. Cette dernière est réalisée à l'aide d'un solvant comme l'eau, d'un alcool de C₁ à C₄, d'une cétone ou d'un mélange eau-alcool C₁ à C₄ ou eau-cétone en toute proportion.

Le rapport plante / solvant varie de 1/3 à 1/20. L'extraction peut être menée sous agitation ou statiquement. La température d'extraction varie de la température d'ébullition du solvant à la température ambiante et sa durée s'étale de 1 heure à 24 heures.

Une fois l'extraction réalisée, les solutions sont récupérées par une séparation solide / liquide adéquate : filtration, décantation, centrifugation, essorage...

Une deuxième extraction peut être menée sur le marc.

Les paramètres entrent alors dans les plages décrites ci-dessus pour la première extraction. Les deux solutions sont alors jointes.

Elles peuvent être utilisées telles quelles ou subir un traitement complémentaire. Pour ce faire, les solutions sont évaporées sous pression réduite à des températures situées entre 40°C et 100°C. cette concentration peut être menée jusqu'à évaporation complète du solvant et obtention d'une poudre après séchage. Une concentration partielle des solutions peut permettre l'utilisation d'un concentrat mais aussi l'obtention d'une phase intermédiaire qui permettra la réalisation d'extrait purifié. Dans ce cas, l'extrait peut être purifié par ajout d'un solvant plus apolaire que le solvant d'extraction, comme les alcools en C₁ et C₄ à haut titre ou une cétone, filtration et récupération du surnageant. Ce surnageant peut être concentré à son tour jusqu'à séchage et l'obtention d'une poudre, s'il y a lieu.

Les extraits sont dosés pour leur teneur en hétérosides stéroliques totaux (HST) par la technique classique par révélation à l'acide sulfurique. Les teneurs en HST varient selon les solvants d'extraction mais aussi selon les étapes de purification éventuelles. Les valeurs se situent alors entre 8 et 40 %. Les HST représentent une famille assez étendue de substances. Le composé majoritaire est le desgluconéoruscoside (entre 8 et 12 % notamment) accompagné de la néoruscine, de la dégluconéoruscine.

Ce procédé d'extraction est applicable aux autres extraits végétaux convenant dans le cadre de la présente invention.

La teneur en extrait de *Ruscus aculeatus* des compositions cosmétiques selon l'invention est avantageusement supérieure à 0,6 % et varie de préférence entre 0,8 et 10 % en poids, avantageusement 1 et 10 %, par rapport à la composition bien que cet intervalle ne soit pas limitatif.

La composition cosmétique, outre ledit extrait de *Ruscus aculeatus* et le véhicule inerte, peut comprendre d'autres principes actifs conduisant à un effet complémentaire ou éventuellement synergique.

Les principes actifs additionnels sont notamment choisis parmi ceux déjà connus et qui ont été cités dans la présentation de l'invention.

On citera notamment les vitamines, les oligo-éléments, les dérivés protéiques, les huiles essentielles, les agents antifongiques, les molécules de synthèse chimique réputées pour leur action spécifique au niveau des récepteurs androgéniques ou sur la synthèse ou l'expression des 5-α réductases.

Bien entendu, cette liste n'est pas limitative et l'homme du métier pourra avec ses connaissances choisir d'autres matières actives qui en complément de la composition cosmétique selon l'invention produiront l'effet souhaité.

Selon le véhicule inerte associé à l'extrait de *Ruscus aculeatus,* la composition cosmétique pourra se présenter sous la forme de lotions, gels, mousses, shampooings, émulsions huile dans l'eau, émulsions eau dans huile, éventuellement bi- ou tri-phasiques.

La composition est généralement une composition aqueuse constituée par de l'eau ou un mélange d'eau et d'un solvant organique utilisé pour solubiliser les composés qui ne seraient pas suffisamment solubles dans l'eau, notamment l'éthanol.

Le pH de la composition appliquée sur les cheveux est de préférence compris entre 3 et 11, avantageusement compris entre 4 et 10. Il est ajusté à la valeur désirée au moyen d'agents alcalinisants habituellement utilisés dans les compositions capillaires.

La composition capillaire peut en outre renfermer au moins un adjuvant utilisé habituellement dans les compositions capillaires.

Parmi ces adjuvants, on peut citer les agents conservateurs, les agents séquestrants, les agents antioxydants, les parfums, les tensioactifs non ioniques, les filtres solaires etc.

La composition cosmétique peut également se présenter sous forme d'une dispersion de particules de polymères filmogènes bien connus. Elle peut également comprendre des plastifiants.

Les plastifiants peuvent être de nature hydrophile ou hydrophobe (ou peu hydrophile) ou des mélanges constitués d'un plastifiant hydrophile et d'un plastifiant hydrophobe.

Parmi les agents plastifiants, on peut citer les éthers de glycol, comme l'éthoxydiglycol ou l'hexylène glycol.

Parmi les agents tensioactifs anioniques qui peuvent être utilisés seuls ou en mélanges, on peut citer en particulier les sels alcalins, les sels d'ammonium, les sels d'amine ou les sels d'amino-alcool des composés bien connus tels que les alcoyles sulfates, les alcoyles éther sulfates, les alcoyles amide sulfates, les alcoyles éther sulfosuccinates.

Parmi les agents tensioactifs nom ioniques qui peuvent être utilisés seuls ou en mélange on peut citer en particulier : les alcools, les alcoylphénols et acides gras polyéthoxylés, polypropoxylés ou polyglycérolés à chaîne grasse comportant 8 à 18 atomes de carbone ou les tensioactifs du type Cremophor RH 40 ®.

Comme épaississant, on peut citer les polysiloxanes. Parmi les agents liants, on peut citer les huiles de silicone volatiles ou non.

La composition cosmétique peut également comprendre une ou plusieurs huiles essentielles telles que l'huile essentielle de romarin, l'huile essentielle de pin ou d'autres huiles végétales.

Selon les formulations, les compositions cosmétiques comprennent un ou plusieurs des composés constituant le véhicule qui sont cités dans les exemples qui vont être décrits ci-après.

L'invention est maintenant décrite en relation avec les exemples ci-après.

### Exemples d'extraction

### Exemple 1

100 kg de rhizomes de ruscus sec sont broyés puis extraits avec 800 litres d'eau à 90°C pendant 3 heures. Les jus d'extraction sont récupérés par essorage puis clarifiés. La solution est concentrée sous pression réduite à 70°C. Le concentrat obtenu est ajusté par addition d'alcool 96 % de façon à obtenir un titre alcoolique situé entre 80 et 90%. un précipité gommeux est alors éliminé par filtration. on récupère un extrait limpide dont la teneur en matière sèche est ajustée entre 8 et 10 % par addition d'alcool 85%.

Les hétérosides stéroliques totaux sont dosés par la technique de l'acide sulfurique entre 15 et 20 % par rapport à la matière sèche. Parmi ces composés, le desgluconéoruscoside est majoritaire avec une teneur se situant entre 9 et 12 % par CLHP.

### Exemple 2

2 kg de rhizomes de ruscus sec sont broyés puis extraits à reflux par 10 kg d'alcool 95 %. Les jus d'extraction sont récupérés par filtration, les marcs sont réextraits avec 8 kg d'alcool 95 %, les jus sont de nouveau récupérés par filtration et joints à ceux de la 1ère extraction. Les solutions réunies sont concentrées, puis séchées sous vide à 50°C. on obtient ainsi une poudre de couleur marron, hygroscopique. Les hétérosides stéroliques totaux, dosés par la technique à l'acide sulfurique, s'élèvent à des valeurs situées entre 10 et 15 %. Le desgluconéoruscoside est le produit majoritaire avec une teneur variant de 8 à 12 % selon CLHP.

### Mise en évidence de l'activité de l'extrait de ruscus

L'extrait végétal est évalué pour son activité stimulante sur la cinétique de production de VEGF par les cellules en culture de la papille dermique folliculaire du cuir chevelu humain.

En effet, les cellules de la papille dermique du follicule pileux présentent des récepteurs à ce facteur et le produisent *in vitro*. La mise en évidence de l'activité de l'extrait végétal est effectuée selon la technique décrite ci-après à partir d'une solution d'extrait végétal à 8,9 % de matière active.

La suspension de cellules de la papille dermique (2.5 x 10⁵ cellules/ 2 ml) dans le milieu DMEM (Dubeico modified eagle medium) contenant du sérum de veau foetal (SVF) est distribuée dans les plaques 6 puits (2.5 x 10⁵ cellules/puits) et incubée pendant 18 heures à 37°C dans une atmosphère à 5 % CO₂. Les cellules de la papille folliculaire sont alors rincées avec un tampon phosphate PBS pour éliminer les cellules non-adhérentes puis exposées aux produits à tester inclus dans du milieu sans SVF.

Une solution mère à 1 % de chaque extrait obtenu selon l'exemple 1 est préparée directement dans le DMEM sans SVF. L'extrait est testé en culture à la concentration 200 µg/ml (dose non cytotoxique).

Les lots suivants ont été réalisés :
- Lot témoin
- 1 solution de ruscus RTH 16 à 200 µg/ml.

Pour chaque lot, 3 puits sont réalisés.

Les milieux de culture de chacun des puits sont récupérés à t + 12 h, t + 24h, t + 48h et t + 72h, centrifugés à 3000 tr/mn et conservés à -80°C.

Après avoir récupéré les surnageants, les cellules sont traitées durant la même cinétique avec du tampon d'extraction (1 ml/puits) composé de Tris-HCI 10 mM, du MgCl₂ 2mM, du NaCl 150 mM, du Triton X100 à 1 % et du PMSF 2 mM (phénylméthylsulfonylfluoride).

Les lysats cellulaires sont ensuite récupérés dans les tubes, soniqués pendant 4 x 15 secondes puis centrifugés à 3000 t/min pendant 15 min à +4°C, cette étape étant nécessaire à l'élimination des débris cellulaires.

Les surnageants sont ensuite conservés à -80°C avant leur utilisation ultérieure pour le dosage du VEGF.

La production du VEGF dans les extraits cellulaires et dans les surnageants est mesurée par Kit Elisa (R&D Biosystems).

Les résultats sont exprimés en picogrammes de VEGF par ml de milieu et en pourcentages d'activité. Les. tableaux (1) et (2) expriment la cinétique de synthèse de VEGF de l'extrait de ruscus RTH 16 dans les extraits cellulaires et les surnageants.

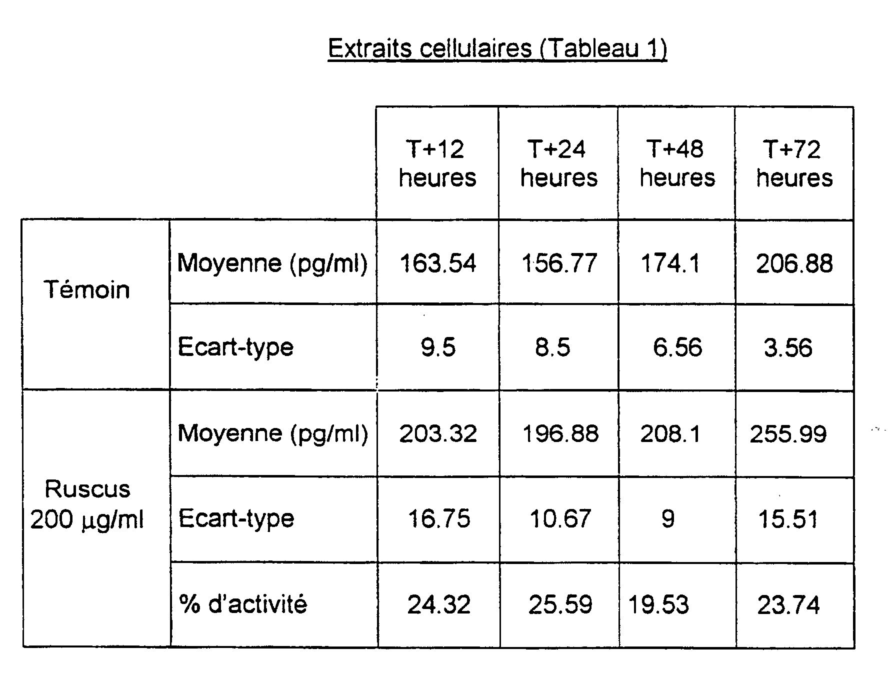

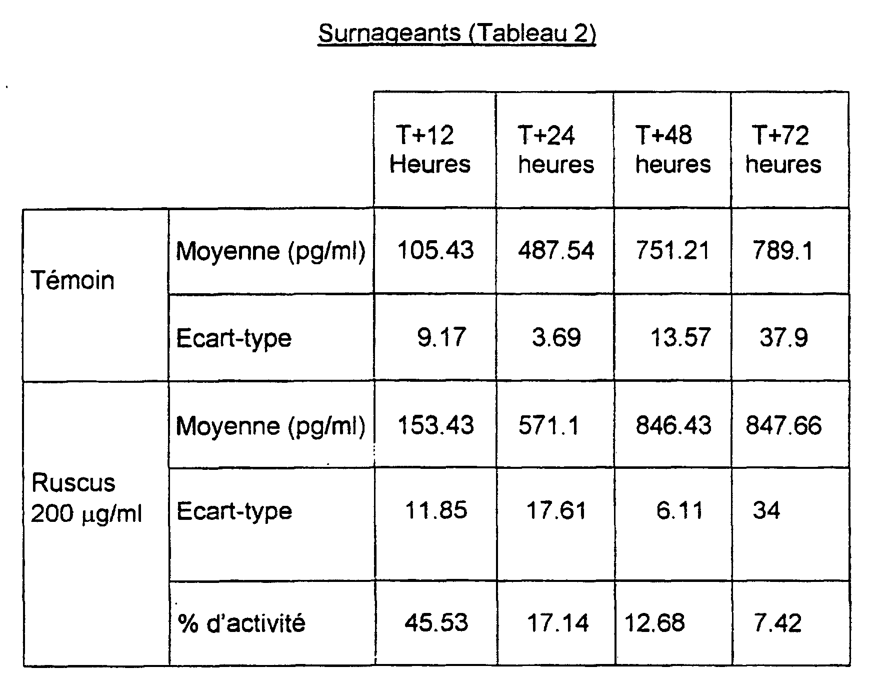

Cette étude a confirmé l'effet stimulant de l'extrait de ruscus RTH 16 à 200 µg/ml durant la cinétique de production du VEGF par les cellules de la papille folliculaire.

Dans les extraits cellulaires, le ruscus RTH 16 est actif durant la cinétique de production du VEGF. Les activités enregistrées ne varient pas de façon significative au cours du temps (+24%, +25.6%, +19.5% et +23.7% pour T+12h, t+24h, t+48h et t+72h respectivement).

Au niveau des surnageants, les activités engendrées par l'extrait de ruscus RTH 16 sur la production du VEGF diminuent avec le temps. Le maximum d'activités est enregistré dans les premières 12 heures (45.5 % d'activité).

A 200 µg/ml (soit 18 µg/ml rapporté à la matière active) le ruscus RTH 16 engendre une stimulation sur la production du VEGF, facteur déterminant de la réponse vasculaire. cette stimulation confirme la potentialité stimulante de l'extrait de ruscus RTH 16 sur la production de ce facteur angiogénique par des cellules de la papille dermique du follicule pileux. D'autre part, le VEGF étant capable de réguler la synthèse de collagène par les cellules de la papille dermique, l'extrait de ruscus RTH 16 paraît un candidat de choix pour prévenir, de manière physiologique, les atteintes matricielles observées localement en cas de chute.

### Exemples de formulation

### Exemple 1 - sérum antichute

| | |
|---|---|
| EXTRAIT DE RUSCUS RTH 16 | 0,6 à 2 % |
| PROVITAMINE B5 | 0,3 % |
| GLUCONATE DE ZINC | 0,10 % |
| PYRIDOXINE CHLORHYDRATE | 0,10 % |
| PHYTOPEPTIDES | 1 % |
| EXTRAIT DE QUINQUINA | 0,75 % |
| ALCOOL 95° | 16 % Vol |
| CREMOPHOR RH 40® | 0,60 % |
| ETHOXYDIGLYCOL | 2 % |
| EAU PURIFIEE QSP | 100 ml |

### Exemple 2 - Sérum antichute bi-phasique

| Phase lipophile | |
|---|---|
| EXTRAIT DE SERENOA REPENS | 0,5 g |
| ESSENCE D'ANETH | 2 g |
| CYCLOMETHICONE D5 | 35 g |

| Phase polaire | |
|---|---|
| HEXYLENE GLYCOL | 10 g |
| DIMETHICONE COPOLYOL | QS |
| EXTRAIT DE RUSCUS RTH 16 | 6 g (3 à 10 g) |
| ALCOOL 95° | 44 ml |
| PARFUM | QS |

### Exemple 3 - lotion capillaire

| | |
|---|---|
| EXTRAIT DE RUSCUS RTH 16 | 0,6 à 2 % |
| PIROCTONOLAMINE | 0,05 % |
| EXTRAIT FLUIDE DE CAPUCINE | 1 % |
| DIMETHICONE COPOLYOL | 0,6 % |
| ESSENCE D'ORANGE | 0,03 % |
| HUILE ESSENTIELLE DE ROMARIN | 0,05 % |
| CHLORURE DE CETRIMONIUM | 0,5 % |
| ALCOOL | 50 % vol |
| EAU PURIFIEE QS | 100 ml |

### Exemple 4 - Solution antichute bi-phasique

| Phase apolaire (20 %) | |
|---|---|
| ACETATE D'ALPHA TOCOPHEROL | 5 g |
| CONCENTRE HUILEUX DE VITAMINE A | 0,6 g |
| GLYCERYL LINOLEATE | 1,1 g |
| EXTRAIT STEROLIQUE DE SABAL | 1,1 g |
| ESSENCE D'ORANGE | 3,2 g |
| ESSENCE DE LAVANDE | 1,1 g |
| ALCOOL ABSOLU | 30 g |
| CYCLOMETHICONE QSP | 100 g |

| Phase polaire (80 %) | |
|---|---|
| PYRIDOXINE CHLORHYDRATE | 0,5 g |
| NICOTINAMIDE | 0,25 g |
| PROVITAMINE B5 | 0,5 g |
| LACTAMIDE MEA | 0,75 g |
| DIMETHICONE COPOLYOL | 0,60 g |
| ETHOXYDIGLYCOL | 3 g |
| SULFATE DE ZINC | 1 g |
| EXTRAIT DE RUSCUS RTH 16 | 3 g |
| ALCOOL ETHYLIQUE | 20 % vol. |
| EAU DEMINERALISEE QSP | 100 g |

### Exemple 5 - Shampooing Antichute

| | |
|---|---|
| EXTRAIT LIPOSTEROLIQUE DE SABAL | 0,3 g |
| EXTRAIT DE RUSCUS RTH 16 | 3 g |
| SALICYLATE DE ZINC | 2 g |
| DODECYL POLYGLUCOSIDE | 4 g |
| LAURYETHER SULFOSUCCINATE 2Na | 9 g |
| COCOAMPHODIACETATE | 1,7 g |
| ALKYLPOLYPEPTIDE DE BLE | 1,2 g |
| POLYMERE DEMELANT | QS |
| ELFACOS GT 282 | QS |
| COCAMIDE MEA | QS |
| PARFUM | QS |
| EAU DEMINERALISEE QSP | 100 g |

Quelques modèles types de formules ont été décrits. Il est évident que la liste n'est pas limitative et qu'elle concerne toute forme galénique à usage capillaire pour le soin du cheveu et du cuir chevelu.

## Revendications

1. Utilisation d'un extrait de *Ruscus aculeatus* pour la fabrication d'une composition pharmaceutique ou cosmétique destinée au traitement des désordres capillaires.

2. Utilisation selon la revendication 1, **caractérisée en ce que** ledit extrait de *Ruscus aculeatus* comprend une teneur efficace en hétérosides stéroliques totaux (HST).

3. Utilisation selon la revendication 2, **caractérisée en ce que** les hétérosides totaux sont choisis parmi le desgluconéoruscoside, la néoruscine, la dégluconéoruscine.

4. Utilisation selon la revendication 3, **caractérisée en ce que** ledit extrait de *Ruscus aculeatus* est obtenu par extraction du broyât à l'aide d'un solvant polaire puis récupération de la solution et concentration éventuelle.

5. Utilisation selon la revendication 4, **caractérisée en ce qu'**après concentration partielle, ledit extrait est purifié par extraction avec un autre solvant plus apolaire que le solvant d'extraction.

6. Utilisation selon la revendication 5, **caractérisée en ce que** ledit extrait de *Ruscus aculeatus* comprend entre 8 et 40 % en poids d'hétérosides stéroliques, dont notamment 8 à 12 % de désgluconéoruscoside.

7. Utilisation selon l'une des revendications précédentes, **caractérisée en ce que** ladite composition contient au moins 0,6 % en poids d'extrait de *Ruscus aculeatus*, de préférence 0,8 à 10 %.

8. Utilisation selon l'une des revendications précédentes, **caractérisée en ce que** ladite composition se présente sous la forme de lotions, gels, mousses, shampooings, émultions huile dans l'eau, émulsions eau dans l'huile, éventuellement bi- ou tri-phasiques.

## Claims

1. Use of an extract of *Ruscus aculeatus* in the manufacture of a pharmaceutical or cosmetic composition intended for the treatment of hair disorders.

2. Use according to Claim 1, **characterized in that** the said extract of *Ruscus aculeatus* comprises an effective content of total sterol heterosides (TSH).

3. Use according to Claim 2, **characterized in that** the total heterosides are chosen from degluconeoruscoside, neoruscin or degluconeoruscin.

4. Use according to Claim 3, **characterized in that** the said extract of *Ruscus aculeatus* is obtained by extraction of the ground material using a polar solvent and then recovery of the solution and optional concentration.

5. Use according to Claim 4, **characterized in that**, after partial concentration, the said extract is purified by extraction with another solvent which is less polar than the extraction solvent.

6. Use according to Claim 5, **characterized in that** the said extract of *Ruscus aculeatus* comprises between 8 and 40% by weight of sterol heterosides, including in particular 8 to 12% of degluconeoruscoside.

7. Use according to one of the preceding claims, **characterized in that** the said composition comprises at least 0.6% by weight of extract of *Ruscus aculeatus*, preferably 0.8 to 10%.

8. Use according to one of the preceding claims, **characterized in that** the said composition is provided in the form of lotions, gels, foams, shampoos, oil-in-water emulsions or water-in-oil emulsions, optionally with two or three phases.

## Patentansprüche

1. Verwendung eines Extrakts von *Ruscus aculeatus* für die Herstellung einer pharmazeutischen oder kosmetischen Zusammensetzung, die zur Behandlung von Haarstörungen bestimmt ist.

2. Verwendung nach Anspruch 1, **dadurch gekennzeichnet, dass** der Extrakt von *Ruscus aculeatus* einen wirksamen Gehalt an sterolischen Gesamt-Heterosiden (STH) enthält.

3. Verwendung nach Anspruch 2, **dadurch gekennzeichnet, dass** die Gesamt-Heteroside ausgewählt sind aus Desgluconeoruscosid, Neoruscin, Degluconeoruscin.

4. Verwendung nach Anspruch 3, **dadurch gekennzeichnet, dass** der Extrakt von *Ruscus aculeatus* durch Extraktion des Zerkleinerungsgutes mit Hilfe eines polaren Lösungsmittels, anschließende Gewinnung der Lösung und gegebenenfalls Konzentration erhalten wird,

5. Verwendung nach Anspruch 4, **dadurch gekennzeichnet, dass** nach teilweiser Konzentration der Extrakt durch Extraktion mit einem anderen Lösungsmittel, das unpolarer ist als das Extraktionslösungsmittel, gereinigt wird.

6. Verwendung nach Anspruch 5, **dadurch gekennzeichnet, dass** der Extrakt von *Ruscus aculeatus* zwischen 8 und 40 Gew.-% sterolische Heteroside, von diesen Insbesondere 8 bis 12 % Desgluconeoruscosid, umfasst.

7. Verwendung nach einem der vorangehenden Ansprüche, **dadurch gekennzeichnet, dass** die Zusammensetzung mindestens 0,6 Gew.-% Extrakt von *Ruscus aculeatus*, vorzugsweise 0,8 bis 10 %, enthält.

8. Verwendung nach einem der vorangehenden Ansprüche, **dadurch gekennzeichnet, dass** die Zusammensetzung in Form von Lotionen, Gelen, Schäumen, Shampoos, gegebenenfalls 2- oder 3-phasigen Öl-in-Wasser-Emulsionen, Wasser-in-Öl-Emulsionen vorliegt.
